# EUROPEAN PATENT APPLICATION

(11) **EP 4 393 449 A1**
(43) Date of publication of application: **03.07.2024**
(21) Application number: 23150054.7
(22) Date of filing: 02.01.2023
(51) Int. Cl.: A61C 19/04, A61B 5/00, G16H 40/63, G16H 50/30, G16H 50/50

(54) **METHOD AND SYSTEM FOR TOOTH WEAR DETECTION**

(71) Applicant: 3Shape A/S, 1060 Copenhagen K (DK)
(72) Inventor: ALEMÃO, João, 1060 Copenhagen K (DK); ALALOUF, Daniella, 1060 Copenhagen K (DK)
(74) Representative: Guardian IP Consulting I/S

(57) **Abstract**

A computer-implemented method for detecting tooth wear on a virtual 3D model (101) of a dental situation is disclosed. The method comprises receiving, by a processor, the virtual 3D model (101) of the dental situation. The method further comprises generating an input (301), from the virtual 3D model (101), for a trained neural network (300) for tooth wear detection, wherein the input (301) comprises at least one input element corresponding to at least a part of the virtual 3D model (101), the at least one input element comprising geometric information of the at least part of the virtual 3D model (101). The method further comprises providing the input (301) to the trained neural network (300) and obtaining an output (305) from the trained neural network (300) based on the provided input (301), wherein the output (305) comprises at least one probability value, wherein the at least one probability value represents a likelihood that the at least one input element corresponds to a tooth wear class. Further, the method comprises assigning the tooth wear class to the at least one input element and registering tooth wear on the at least part of the virtual 3D model (101) based on the assigned tooth wear class to the at least one input element. The at least part of the virtual 3D model (101) is displayed with the registered tooth wear.

## Description

### Technical field

The present disclosure relates to a computer-implemented method and system for detecting tooth wear on a virtual 3D model of a dental situation.

### Background

Tooth wear is a dental condition characterizing loss of tooth substance. It is often painful and impairs the function of teeth. Three types of tooth wear occurring are abrasion, attrition, and erosion. Abrasion is manifested as physical wear of teeth caused by a factor other than tooth-to-tooth contact, such as inappropriate toothbrushing. Attrition is loss of tooth substance from tooth-to-tooth contact. Dental erosion is dissolving of tooth enamel due to the presence of acids in the mouth.

Damage caused by tooth wear is irreversible and can be difficult to repair. Timely detection and monitoring of tooth wear by general practitioners are therefore essential for preserving tooth structure.

Clinical detection and diagnosis of tooth wear are currently based on direct visual inspections of teeth, which are very subjective. Visual inspections are characterized by low sensitivity and low reproducibility when performed by general practitioners. Furthermore, tooth substance loss is visually detectable only when a significant amount of hard dental tissue is already lost.

Digital dentistry and use of intraoral scanners enabled more accurate dental condition detection methods to be developed, thereby reducing, or completely removing, practitioner's subjectivity. For example, it is now possible to acquire two intraoral scans of a patient's dental situation at different time periods. It is then possible to detect tooth wear by comparing the geometries of the two scans and identifying quantifiable geometric differences. The drawback of this method is that tooth wear cannot be determined from only a single scan but instead two scans of the dental situation at different time points are needed.

There is a clear need to develop methods and systems which will aid in tooth wear detection from one single intraoral scan of the patient's dental situation.

### Summary

Disclosed herein is a computer-implemented method for detecting tooth wear on a virtual 3D model of a dental situation comprising:
- receiving, by a processor, the virtual 3D model of the dental situation,
- generating an input, from the virtual 3D model, for a trained neural network for tooth wear detection, wherein the input comprises at least one input element corresponding to at least a part of the virtual 3D model, the at least one input element comprising geometric information of the at least part of the virtual 3D model,
- providing the input to the trained neural network,
- obtaining an output from the trained neural network based on the provided input, wherein the output comprises at least one probability value, wherein the at least one probability value represents a likelihood that the at least one input element corresponds to a tooth wear class,
- assigning the tooth wear class to the at least one input element,
- registering tooth wear on the at least part of the virtual 3D model based on the assigned tooth wear class to the at least one input element,
- displaying the at least part of the virtual 3D model with the registered tooth wear.

The method according to an embodiment may comprise receiving, by the processor, the virtual 3D model of the dental situation.

Expression "3D" throughout the present disclosure refers to term "three-dimensional". Similarly, term "2D" refers to term "two-dimensional". Term "virtual 3D model of a dental situation" refers to a virtual, three-dimensional, computer-generated representation of the patient's dental situation. Such virtual 3D model may be constructed, by the processor, based on scan data collected in an intraoral scanning process in which an intraoral scanner is used to scan the patient's dental situation comprising teeth and gingiva. The virtual 3D model can alternatively be generated by using a conventional 3D scanner, also called a lab scanner or a desktop scanner, to scan a gypsum model of the patient's dental situation. The virtual 3D model can be stored in a memory of a computer system, for example in a Standard Triangle Language (STL) format.

The virtual 3D model can be received or accessed by the processor. The virtual 3D model may usually be displayed on a display screen in a form of a 3D mesh, a point cloud, a volumetric representation, or any other suitable 3D representation form.

The virtual 3D model of the dental situation may be obtained from scan data of a single scan of the dental situation and may be used in the method according to the disclosure. This means that tooth wear, with the use of the trained neural network, can be detected on just one scan of the patient's dental situation. In particular, it is not necessary to obtain a second scan at a later point in time and to compare geometries of the two scans. The single scan of the dental situation may include one or more corrective scans for the purpose of accurately constructing the virtual 3D model.

Further, the method may comprise generating the input, from the virtual 3D model, for the trained neural network for tooth wear detection, wherein the input may comprise the at least one input element corresponding to the at least part of the virtual 3D model. The at least one input element may comprise geometric information of the at least part of the virtual 3D model.

The method may make use of the trained neural network to detect presence of tooth wear on the virtual 3D model. For that purpose, the input for the trained neural network may be generated from the virtual 3D model. Depending on the type of the trained neural network, the input may be in 3D format or in 2D format. For example, one type of the trained neural network may be capable of processing 2D images of the virtual 3D model while another type of the trained neural network may be capable of processing the virtual 3D model in form of the point cloud. Thus, the effect of generating step is that the virtual 3D model is adapted for processing by the trained neural network.

In an embodiment, the input may be in 3D format. An example of the input in 3D format may be a point cloud, a 3D mesh, a volumetric representation or a graph representation of the virtual 3D model. It may be possible to transform the input from one 3D representation type, for example from the mesh representation, into another 3D representation type, for example the point cloud representation. A type of the trained neural network capable of processing the input in 3D format may be a trained PointNet network, or a PointNet-type network. These neural networks may be capable of processing the virtual 3D model, or the at least part of the virtual 3D model, in the point cloud form.

The at least one input element may be a point from the point cloud representation of the virtual 3D model. Similarly, the at least one input element may be a facet from the mesh representation of the virtual 3D model or a voxel from the volumetric representation of the virtual 3D. At least one of these input elements may be comprised in the input for the trained neural network. It may also be possible to provide all of the input elements corresponding to the virtual 3D model into the trained neural network as the input. For example, it may be possible to provide all points from the point cloud representation of the virtual 3D model into the trained neural network.

Generating the input may comprise sampling the at least one input element or converting the at least one input element into a numerical form. For example, one or more points from the point cloud representation of the virtual 3D model may be converted into numerical form, for example a matrix of input values. The matrix may then form the input for the trained neural network.

If the virtual 3D model is in the 3D mesh representation, it may be possible to convert the virtual 3D model into the point cloud representation, by collapsing all vertices from the 3D mesh representation so that only points remain, thus forming the point cloud. The remaining point cloud may then be processed by the PointNet network, or the PointNet-type network.

In another embodiment, the input may be in 2D format such as a 2D image or a plurality of 2D images of at least one tooth surface of at least one tooth in the virtual 3D model.

Generating the input, from the virtual 3D model, for the trained neural network for tooth wear detection may comprise generating one or more 2D images of the at least one tooth surface of the at least one tooth in the virtual 3D model. The one or more 2D images may then form the at least one input element. Alternatively, the at least one input element may be a pixel belonging to the one or more 2D images. The one or more 2D images may be generated by means of a virtual camera, as will be explained later.

Geometric information, also referred to as topological information, comprised in the at least one input element may be a depth information relating to a camera position, an angle between a facet normal and a camera direction, the facet normal and/or a curvature information. The term "camera" in this context relates to the virtual camera used to view the virtual 3D model. The camera may be characterized by a virtual camera property, for example field of view (FOV) and/or focal length.

The depth information may comprise distance information of the at least part of the virtual 3D model to the camera. Depth information may be in the form of a depth image. The depth image may display the at least part of the virtual 3D model according to its distance from the camera. For example, parts of the virtual 3D model more distant to the camera may be shaded darker than the parts closer to the camera. A distance threshold may be defined such that, when a distance between the camera and the part of the virtual 3D model is greater than the distance threshold, the part of the virtual 3D model is not shown in the depth information. The distance of the at least part of the virtual 3D model from a given camera position indirectly describes a topology of the at least part of the virtual 3D model, and consequently a smoothness of a surface of the at least part of the virtual 3D model. The variations of surface smoothness may represent a relevant parameter in detecting tooth wear in the at least part of the virtual 3D model.

The facet normal may be a vector perpendicular to a facet of the virtual 3D model. The facet can be seen as a "building block" of the virtual 3D model in 3D mesh format and can, for example, be a triangle if the virtual 3D model is in format of a triangular mesh. A triangular facet may be defined by three points (vertices) interconnected with three edges. Two neighboring facets that have a small variation in their respective facet normals may indicate a local area that is smooth. However, if a significant variation in the facet normals can be observed, then the neighboring facets define non-smooth surface which is relevant for tooth wear detection. The variation in two surface normals can be expressed in terms of an angle between the two surface normals.

The curvature information may be in the form of a per-facet value and may be obtained by measuring a change along facet normals of neighboring facets of the virtual 3D model. This change may give insight into whether the associated section of the virtual 3D model is flat or curved. Thereby, the curvature information may describe topology of teeth by revealing smooth and non-smooth surfaces. Tooth wear may be characterized by an abrupt change of relative curvature between areas in teeth surfaces. Tooth wear may also be associated with a localized smoothness (regions of low curvature) in certain areas. The curvature information is thus a significant parameter in detecting tooth wear, detecting locations of tooth wear lesions, tooth wear types and/or tooth wear severity.

Once generated, the input may be provided to the trained neural network in order to obtain the output from the trained neural network. All information forming the input may be concatenated into a final input tensor.

In an embodiment, generating the input, from the virtual 3D model, for the trained neural network may comprise generating one or more two-dimensional (2D) images of the at least one tooth surface of the at least one tooth in the virtual 3D model.

The trained neural network may thus be suitable to processes 2D input format. The one or more of 2D images may be generated by the virtual camera, referred to also as the camera. The virtual camera may take snapshots of the individual teeth of the virtual 3D model. For example, a tooth in the segmented 3D model may be positioned such that a desired tooth surface of the tooth is facing the camera. The tooth may be rotated and translated via a local coordinate system such that one or more of the tooth surfaces are photographed. This may be an automatic process where predetermined movements of the tooth have been set up in order to capture, via the virtual camera, the one or more of the tooth surfaces. Alternatively, the virtual camera may be moved to a plurality of predetermined positions to capture snapshots of the desired tooth surface. The snapshots of the individual teeth may alternatively be generated by manual movement of the virtual camera to desired positions.

The at least one tooth in the virtual 3D model may be obtained by segmenting the virtual 3D model into a plurality of teeth. Each tooth may comprise a plurality of tooth surfaces. The plurality of tooth surfaces for each tooth may comprise a buccal surface, a lingual surface, a distal, a mesial, and/or an occlusal surface. The plurality of tooth surfaces may also comprise a cervical and/or an incisal surface of the tooth. The lingual surface of the at least one tooth may be a surface facing a tongue and may be nearest to the tongue. The buccal surface may face the cheek. The occlusal surface may be a chewing surface. The occlusal surface may also be the incisal surface, referring to a biting edge of incisal teeth. The cervical surface may constitute a boundary between enamel covering a crown and a cementum covering a root.

Segmenting the virtual 3D model may be performed via a segmentation process which allows for identification of distinct dental objects such as individual teeth and/or surrounding gingiva in the virtual 3D model. Individual teeth can be assigned a tooth identifier, for example according to the Universal Numbering Notation (UNN) in which numerals 1-32 are assigned to human teeth. The segmentation process may comprise use of algorithms such as Principal Component Analysis (PCA) or harmonic fields. The segmentation process may alternatively or additionally comprise use of machine learning models.

Optionally, providing the input to the trained neural network may comprise providing semantic information of the at least part of the virtual 3D model to the trained neural network. Thus, the input may additionally comprise semantic information of the at least part of the virtual 3D model.

Semantic information of the at least part of the virtual 3D model may be the tooth identifier, a tooth surface identifier, and/or a jaw identifier. The tooth surface identifier may provide information on the type of surface in the input (e.g. a buccal, lingual or occlusal surface) while the jaw identifier may provide information to what jaw (upper or lower) the input or the at least one input element represents.

By incorporating semantic information into the input to the trained neural network, a semantic context may be given to data present in the input, thereby aiding the trained neural network in processing of the geometric information. Semantic information may be converted to numerical format, as other data formats, before being processed by the trained neural network. A better performance of the trained neural network may be achieved by additionally incorporating this semantic information into the input. For example, tooth wear lesions may occur in a common manner among same or similar kinds of teeth. To detect these tooth wear lesions more efficiently, the trained neural network may use provided additional teeth identifiers as semantic information.

Optionally, providing the input to the trained neural network may comprise providing color information such as a Red-Green-Blue (RGB) intensity value of the at least one input element. Thereby, color information of the at least one input element may be a part of the input to the trained neural network. Presence of the color information may be beneficial as it facilitates identifying tooth wear lesions where dentine may be exposed, because dentine is more yellow compared to enamel. This is particularly beneficial in cases of severe tooth wear which is characterized by tooth substance enamel loss. Similarly, once enamel gets eroded, a leftover enamel section that becomes visible is of a different color due to absence of any markings or colorations that may occur due to tea or coffee stains, for example. The difference in color may indicate a tooth wear lesion. Thus, the color information may be of high relevance for the trained neural network in a process of detecting tooth wear lesions, particularly those tooth wear lesions with exposed dentine.

Further, the method may comprise obtaining an output from the trained neural network based on the provided input, wherein the output comprises the at least one probability value, wherein the at least one probability value represents the likelihood that the at least one input element corresponds to the tooth wear class.

The tooth wear class may indicate presence of tooth wear on the at least one input element, for example the tooth wear class may be one of "Yes" or "No" to indicate presence of tooth wear.

In another example, the tooth wear class may be a tooth wear severity value, indicating how severe detected tooth wear lesion is .

In yet another example, the tooth wear class may be a tooth wear type, indicating a specific type of the detected tooth wear lesion. As mentioned previously, the tooth wear type may be one of attrition, abrasion and dental erosion.

Based on the obtained at least one probability value, the tooth wear class may be assigned to the at least one input element. For example, if the at least one input element is a specific pixel, and the at least one probability value representing a likelihood that the specific pixel corresponds to the tooth wear class "Yes" is 95%, then the tooth wear class "Yes" is assigned to that specific pixel. Thereby, the at least one input element may be assigned the tooth wear class having a highest probability value. As mentioned, the tooth wear class "Yes" indicates presence of tooth wear on the specific pixel.

Based on the assigned tooth wear class to the at least one input element, tooth wear may be registered on the at least part of the virtual 3D model. As mentioned, the at least one input element corresponds to the at least part of the virtual 3D model. In this way, tooth wear is associated with the at least part of the virtual 3D model. Correspondence of the at least one input element and the at least part of the virtual 3D model may be established by means of an identifier. For example, the at least one input element may be a point with the identifier which defines position of the point in the virtual 3D model. The same principle may be applicable to other type of input elements such as vertices, facets, voxels, pixels.

Once tooth wear is registered, the at least part of the virtual 3D model may be displayed. In this way the information on tooth wear presence is conveyed to a user. For example, the at least part of the virtual 3D model may be highlighted to visually distinguish it from the rest of the virtual 3D model. Additionally or alternatively, the at least part of the virtual 3D model may be colored in a different color, or it may be encircled to visually distinguish the at least part of the virtual 3D model from the rest of the virtual 3D model. Alternatively, a line defining a border of the at least part of the virtual 3D model and the rest of the virtual 3D model may be highlighted.

According to an embodiment of the disclosure, the tooth wear class may be a tooth wear severity value. In this embodiment the trained neural network may perform a classification task and may classify the at least one input element, for example the one or more 2D images of tooth surfaces of the at least one tooth, into a corresponding tooth wear severity value. Thereby, the tooth wear severity value may be assigned for each tooth surface of the at least one tooth. The tooth surfaces of the at least one tooth in the virtual 3D model may be, as mentioned previously, the lingual, the buccal, the mesial, the distal and/or the occlusal surface of the tooth.

There may be several tooth wear severity values defined. In one example, the tooth wear severity value may correspond to values of a clinically valid tooth wear index, such as the Tooth Wear Index (TWI). Generally, the TWI comprises a five-point ordinal scale, scores of zero to four (0 - 4), to grade various tooth surfaces. For example, the TWI score four signifies the most severe tooth wear condition, the complete loss of enamel or pulp exposure. Instead of the TWI, the Lussi index, Basic Erosive Wear Examination (BEWE) index may also be used to define the tooth wear severity value. The advantage of using the clinically valid tooth wear index is that the tooth wear indication can easily be interpreted by the user.

The trained neural network may, in a manner described above, perform a classification task to classify the at least one input element, for example the one or more 2D images of the at least one tooth surface of the at least one tooth in the virtual 3D model, according to clinically accepted criteria for tooth wear into an associated tooth wear severity value.

The output from the trained neural network may be in a vector format of fixed size which can be derived from a compact representation of the input. Values in the output may comprise the at least one probability value representing the likelihood that the at least one input element corresponds to the tooth wear class.

In an embodiment, the at least one input element with the assigned tooth wear class may indicate a location of a tooth wear lesion on the virtual 3D model. The output from the trained neural network may thus indicate an area on the virtual 3D model corresponding to a part of the dental situation worn down due to tooth wear. In this case, the trained neural network may perform a semantic segmentation task in which, for the at least one input element being a pixel, a facet or a point, the tooth wear class may be assigned. The effect of performing the semantic segmentation task is therefore to identify an exact location and size of the tooth wear lesion on the virtual 3D model.

Obtaining the output from the trained neural network may, in an embodiment, comprise obtaining a segmentation map.

The segmentation map may be used to assign the tooth wear class to the at least one input element. For example, a pixel of the input 2D image may be assigned the tooth wear class. This assignment may be achieved by overlaying the input, for example the 2D image, with a segmentation mask.

The tooth wear class may be a predefined numerical value. For example, value "one" (1) may indicate tooth wear, value "two" (2) may indicate the tooth surface and value "three" (3) may indicate gingiva.

The segmentation mask may indicate a location of a structure of interest within the input, for example within the input 2D image. The segmentation mask may be a 2D vector that is of same size as the input. The segmentation mask has therefore same number of spatial elements as the input and represents a reconstruction of the input. Each value of the segmentation mask may be a tooth wear class. For example, for a given pixel or a facet of the input, the trained neural network may assign the tooth wear class. For example, the tooth wear class may have a value "one", to indicate presence of tooth wear. The tooth wear class may have a value "two" to indicate tooth surface or value "three" to indicate gingiva. In this way, the at least one input element, for example a pixel, a point, a voxel or a facet of the input may be assigned the corresponding tooth wear class.

In one example, the output from the trained neural network may be reconstructed to the 2D format, such as a 2D image, which may be overlaid, or projected, over the virtual 3D model.

In an example, displaying step may comprise displaying a tabular overview showing each tooth surface of the at least one tooth in the virtual 3D model and the corresponding tooth wear severity value. This tabular overview may be in a form of a dental chart. Alternatively or additionally, the tooth wear severity value may be displayed directly on the virtual 3D model, for example adjacent to associated tooth surface.

According to an embodiment, the trained neural network may be a trained convolutional neural network (CNN). The trained CNN may comprise a plurality of convolutional layers capable of capturing low-level features of the input, i.e. obtaining convolved features. Moreover, one or more of pooling layers may be present, responsible for reducing the spatial size of convolved features. Convolutional neural networks may be particularly suitable for processing matrix information such as 2D images.

Another example of the trained neural network, particularly suitable for performing classification tasks, is a trained Residual Network (ResNet) or a variation of it.

Example of the trained neural network suitable for performing semantic segmentation tasks is a trained U-Net or a variant of it, for example U-Net++.

In an alternative embodiment, the trained neural network may be a trained transformer network. Transformer networks may be suitable for processing matrix information such as 2D images and have similar performance to convolutional neural networks.

In case of three-dimensional (3D) format of the input, such as a point cloud, a suitable neural network architecture may be a PointNet network or a variation of it. The PointNet network is capable of processing input in the 3D format, such as a point cloud.

The tooth wear class may be, alternatively or additionally, a tooth wear type.

Three different tooth wear type classes may be defined, such as abrasion, attrition and dental erosion. The trained neural network may classify the at least one input element according to the defined tooth wear types. The at least one input element in this case may be the 2D image or the plurality of 2D images of the at least one tooth surface of the at least one tooth in the virtual 3D model.

In one embodiment, the tooth wear class may comprise the tooth wear severity value and the tooth wear type. The trained neural network may, in this case, perform two simultaneous classification tasks. In this case, the trained neural network may be of a multi-task learning architecture. In this type of architecture, different branches may be employed for determining the tooth wear severity value and the tooth wear type in the input. The trained neural network may, alternatively, comprise two different trained neural networks. The tooth wear severity value may be assigned to the at least one input element based on an output from a first trained neural network. The tooth wear type may be assigned to the at least one input element based on an output from a second trained neural network.

In an embodiment, the tooth wear class may comprise the location of the tooth wear lesion. In this way, clinical utility of the method according to the disclosure is increased because the tooth wear lesions can be pointed out virtual 3D model.

The tooth wear lesion may be the area worn down due to tooth wear. The trained neural network may be capable of performing the semantic segmentation task to indicate the area worn down due to tooth wear. This may be achieved by generating the segmentation mask indicating where specific structures of interest are located within the input, for example within the 2D image. In this way the trained neural network can detect where the tooth wear is present within the input.

In an embodiment, displaying the at least part of the virtual 3D model with the registered tooth wear may comprise displaying the tooth wear lesion. In this way, the user is visually presented with a tooth wear indication visible on the virtual 3D model. This is of specific benefit to the user as it allows the tooth wear to be detected, quantified, and presented, thereby avoiding subjective visual inspections.

In an embodiment, obtaining the output from the trained neural network may comprise obtaining a probability matrix, wherein the probability matrix allows assignment of the tooth wear class for the at least one input element of the virtual 3D model.

In one embodiment, the tooth wear class may comprise the tooth wear severity and the location of the tooth wear lesion. The trained neural network may, in this case, perform the classification task and the semantic segmentation task. For this purpose, the trained neural network may comprise two different trained neural networks wherein the tooth wear severity may be assigned to the at least one input element based on the output of the first trained neural network performing the classification task. Additionally, the location of the tooth wear lesion may be determined based on an output of a third trained neural network performing the semantic segmentation task.

According to an embodiment of the disclosure, the method may further comprise determining a distribution of the tooth wear class in the input and selecting outliers in the determined distribution. Based on the selected outliers, the tooth wear indication may be identified and displayed. For example, the distribution of the tooth wear class in the input tooth surfaces may comprise surfaces classified with the tooth severity value 4.

In this way a custom reference point on a state of tooth wear may be determined. The custom reference point on the state of tooth wear may also comprise characteristics of the patient such as age, gender, ethnicity and/or region of residence.

Selection of outliers may be based on Linear Discriminant Analysis (LDA), Principal Component Analysis (PCA) or similar methods.

Furthermore, a data processing apparatus comprising means for carrying out the method of any one or more of the presented embodiments is disclosed.

The data processing apparatus may be configured to carry out a method for detecting tooth wear on the virtual 3D model of the dental situation, wherein the method comprises steps of:
- receiving, by the processor, the virtual 3D model of the dental situation;
- generating the input, from the virtual 3D model, for the trained neural network for tooth wear detection, wherein the input comprises the at least one input element corresponding to the at least part of the virtual 3D model, the input element comprising geometric information of the at least part of the virtual 3D model;
- providing the input to the trained neural network;
- obtaining the output from the trained neural network based on the provided input, wherein the output comprises the at least one probability value, wherein the at least one probability value represents the likelihood that the at least one input element corresponds to the tooth wear class;
- assigning the tooth wear class to the at least one input element;
- registering tooth wear on the at least part of the virtual 3D model based on the assigned tooth wear class to the at least one input element;
- displaying the at least part of the virtual 3D model with the registered tooth wear.

A computer program product is further disclosed. The computer program product according to an embodiment may comprise instructions which, when the program is executed by a computer, causes the computer to carry out the method of any one or more of presented embodiments.

The computer program product may comprise instructions which, when the program is executed by a computer, causes the computer to carry out a method for detecting tooth wear on the virtual 3D model of the dental situation, wherein the method comprises steps of:
- receiving, by the processor, the virtual 3D model of the dental situation;
- generating the input, from the virtual 3D model, for the trained neural network for tooth wear detection, wherein the input comprises the at least one input element corresponding to the at least part of the virtual 3D model, the input element comprising geometric information of the at least part of the virtual 3D model;
- providing the input to the trained neural network;
- obtaining the output from the trained neural network based on the provided input, wherein the output comprises the at least one probability value, wherein the at least one probability value represents the likelihood that the at least one input element corresponds to the tooth wear class;
- assigning the tooth wear class to the at least one input element;
- registering tooth wear on the at least part of the virtual 3D model based on the assigned tooth wear class to the at least one input element;
- displaying the at least part of the virtual 3D model with the registered tooth wear.

Further, the disclosure comprises a non-transitory computer readable medium. The non-transitory computer readable medium may comprise instructions which, when executed by a computer, cause the computer to carry out the method of any one or more of presented embodiments.

The non-transitory computer readable medium may comprise instructions which, when executed by a computer, cause the computer to carry out a method for detecting tooth wear on the virtual 3D model of the dental situation, wherein the method comprises steps of:
- receiving, by the processor, the virtual 3D model of the dental situation;
- generating the input, from the virtual 3D model, for the trained neural network for tooth wear detection, wherein the input comprises the at least one input element corresponding to the at least part of the virtual 3D model, the input element comprising geometric information of the at least part of the virtual 3D model;
- providing the input to the trained neural network;
- obtaining the output from the trained neural network based on the provided input, wherein the output comprises the at least one probability value, wherein the at least one probability value represents the likelihood that the at least one input element corresponds to the tooth wear class;
- assigning the tooth wear class to the at least one input element;
- registering tooth wear on the at least part of the virtual 3D model based on the assigned tooth wear class to the at least one input element;
- displaying the at least part of the virtual 3D model with the registered tooth wear.

### Brief description of the figures

Embodiments of the disclosure may be best understood from the following detailed description taken in conjunction with the accompanying figures. The figures are schematic and simplified for clarity, and they just show details to improve the understanding of the claims, while other details are left out. The individual features of each embodiment may each be combined with any or all features of other embodiments. These and other embodiments, features and/or technical effects will be apparent from and elucidated with a reference to the illustrations described hereinafter in which:
FIG. 1 illustrates a virtual 3D model of a dental situation.
FIG. 2 is a flow chart illustrating a method according to an embodiment.
FIG. 3 illustrates a trained neural network used in the method according to an embodiment.
FIG. 4 illustrates a displayed part of the virtual 3D model with highlighted multiple teeth affected by tooth wear according to an embodiment of invention.
FIG. 5 illustrates a displayed part of the virtual 3D model with highlighted tooth wear on two tooth surfaces of a single tooth according to an embodiment of invention.
FIG. 6 illustrates a displayed part of the virtual 3D model with highlighted tooth wear lesions on multiple teeth according to an embodiment of invention.
FIG. 7 illustrates a dental scanning system.

### Detailed description

In the following description, reference is made to the accompanying figures, which show by way of illustration how the invention may be practiced.

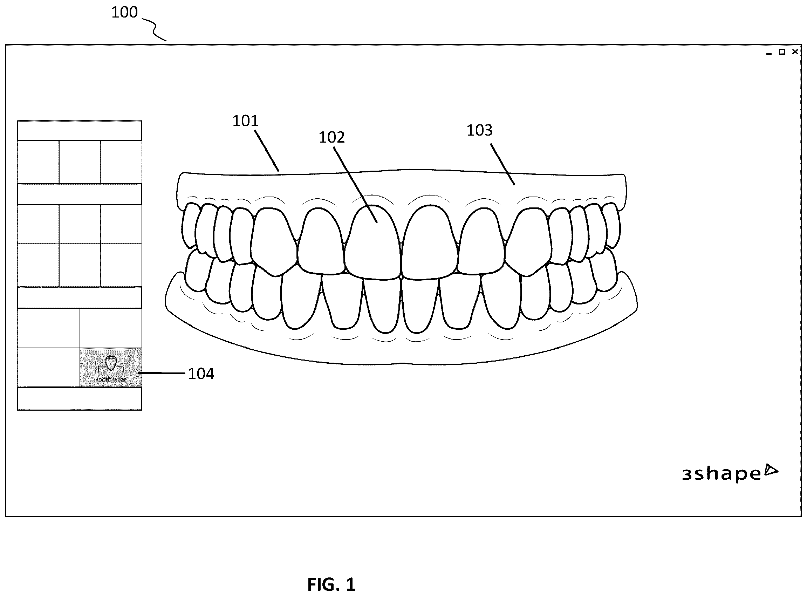

FIG. 1 illustrates a user interface 100 with a displayed virtual 3D model 101 of a dental situation of a patient. The virtual 3D model 101 may be displayed on a display screen in a form of a 3D mesh, a point cloud, a 3D graph, a volumetric representation, or any other suitable 3D representation form. The 3D model 101 may be representative of the dental situation of the patient, i.e. the 3D model 101 may comprise combined representations of teeth and gingiva of the patient's dental situation. The user interface 100 may comprise a button 104 which, once engaged by a user, initiates the method for analysis of the virtual 3D model 101 for identification of tooth wear presence. The method may also be initiated automatically, without user engagement.

To identify and separate individual teeth 102 and gingiva 103 within the virtual 3D model 101, the virtual 3D model 101 may be segmented. This means that facets, for example facets of the 3D mesh representation of the virtual 3D model, belonging to individual teeth 102 as per Universal Numbering System/Notation (UNN), may be determined. In this way individual teeth 102, or parts of the individual teeth 102, can be analyzed for presence of tooth wear. Segmentation of the virtual 3D model 101 is an optional step, and the complete virtual 3D model 101 may instead be analyzed for presence of tooth wear.

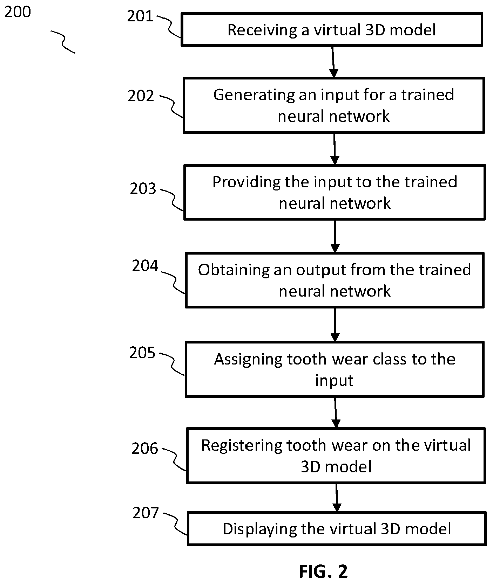

Figure 2 shows a flowchart illustrating a method 200 according to an embodiment.

First, the virtual 3D model 101 may be received, by the processor, in step 201. The virtual 3D model 101 can be stored in a memory of a computer system, for example in a Standard Triangle Language (STL) format. The virtual 3D model 101 can be received by the processor, for example when the user engages the button 104 in the user interface 100. The virtual 3D model 101 may usually be displayed on a display screen in a form of the 3D mesh, the point cloud, the 3D graph, the volumetric representation, or any other suitable 3D representation form.

Step 202 of the method 200 illustrates generating an input, from the virtual 3D model 101, for a trained neural network for tooth wear detection. The input may comprise at least one input element corresponding to at least a part of the virtual 3D model 101. The at least one input element may comprise geometric information of the at least part of the virtual 3D model 101.

The input for the trained neural network may be in 2D or 3D format. For example, the input may comprise one or more 2D images of at least one tooth surface of the at least one tooth 102 in the virtual 3D model 101. In an example, the input may comprise one or more pixels of the one or more 2D images. In another example, the input may comprise one or more points of the point cloud representing the virtual 3D model 101, or one or more facets of the 3D mesh representation of the virtual 3D model 101.

Any of the above may be referred to as input elements. Every input element may correspond to a part of the virtual 3D model 101, and this correspondence may be defined by input element identifiers. For example, the one or more points of the point cloud representing the virtual 3D model 101 may have an identifier defining the location of the one or more points in the point cloud.

The one or more of 2D images may be generated by a virtual camera, referred to also as a camera, taking snapshots of the individual tooth 102 of the virtual 3D model 101. For example, the tooth 102 in the virtual 3D model 101 may be positioned such that a desired tooth surface of the tooth 102 is facing the camera. The tooth 102 may be rotated and translated via a local coordinate system such that one or more of the tooth surfaces may be photographed. This may be an automatic process where predetermined movements of the tooth 102 have been set up in order to capture, via the virtual camera, the one or more of the tooth surfaces.

The individual tooth 102 may be identified by segmenting the virtual 3D model 101 into a plurality of individual teeth 102 and gingiva 103. Each tooth 102 from the plurality of individual teeth may comprise a plurality of tooth surfaces.

Segmentation of the virtual 3D model 101 may be done in several ways. According to an example, segmenting may comprise use of surface curvatures to identify boundaries of tooth representations. A curvature threshold value can be selected to distinguish tooth boundary regions from the rest of surface.

In another example, segmentation of the virtual 3D model 101 may comprise use of a harmonic field to identify tooth boundaries. On the virtual 3D model 101, a harmonic field may be a scalar attached to each mesh vertex satisfying the condition: ΔΦ=0, where Δ is Laplacian operator, subject to Dirichlet boundary constraint conditions. Above equation may be solved, for example using least squares method, to calculate the harmonic field. Segmented teeth 102 can then be extracted by selecting optimal isolines connecting datapoints with same value, as tooth representation boundaries.

In yet another example, segmenting the virtual 3D model 101 may comprise use of a segmentation machine learning model. In particular, the virtual 3D model 101 may be converted into a series of 2D virtual images. The segmentation machine learning model may be applied to the series of 2D virtual images. For each 2D virtual image, segmentation can be performed to distinguish between different teeth classes and gingiva. After classification of each element of each 2D virtual image, backprojection onto the virtual 3D model 101 may be performed. This method for segmenting the virtual 3D model 101 may be advantageous as the segmentation machine learning model utilizes the series of 2D virtual images, overall resulting in fast and accurate facet classification.

Geometric information comprised in the at least one input element may be a depth information relating to a camera position, an angle between a facet normal and a camera direction, the facet normal and/or a curvature information.

Step 203 represents providing the input to a trained neural network 300 for tooth wear detection. The trained neural network 300 may be suitable for processing the input, whether the input is in 2D or 3D format. Presence of tooth wear in the input, representing the at least part of dental situation, is identified by applying the trained neural network 300 to the generated input.

Step 204 of the method 200 represents obtaining an output from the trained neural network 300 based on the provided input, wherein the output may comprise at least one probability value, wherein the at least one probability value represents a likelihood that the at least one input element corresponds to a tooth wear class.

In an example, the tooth wear class may be a tooth wear severity value. In a further example, the tooth wear class may be an indication of tooth wear presence on the at least one input element. For example, the indication of tooth wear presence can be "Yes", to indicate positive tooth wear presence, or "No", to indicate negative tooth wear presence, also referred to as absence of tooth wear, on the at least one input element. The tooth wear class may be, in yet a further example, a tooth wear type. The tooth wear class may be a combination of any of the indication of tooth wear presence, the tooth wear severity value and/or the tooth wear type.

With the obtained output, it may be possible to assign the tooth wear class to the at least one input element. This is illustrated in step 205 of the method 200. The at least one input element may therefore be assigned with the tooth wear class having a highest probability value in the output.

Step 206 of the method 200 illustrates registering tooth wear on the at least part of the virtual 3D model 101 based on the assigned tooth wear class to the at least one input element. In this way, tooth wear may be associated with the at least part of the virtual 3D model 101. Correspondence of the at least one input element and the at least part of the virtual 3D model 101 may be established by means of an identifier. For example, the at least one input element may be a point with the identifier which defines position of the point in the virtual 3D model 101. The same principle may be applicable to other type of input elements. The identifier associated with the at least one input element allows for allocation of tooth wear to the at least part of the virtual 3D model 101 corresponding to a part of the dental situation affected by tooth wear.

Step 207 of the method 200 represents displaying the at least part of the virtual 3D model 101 with the registered tooth wear.

The at least part of the virtual 3D model 101 may be displayed on a display screen. Additionally or alternatively, the displaying may comprise playing an audio signal when tooth wear is detected. With the displaying step, the at least part of the virtual 3D model 101, with registered tooth wear, may be pointed out to the user. The user may learn that tooth wear is present on the at least one tooth 102 in the virtual 3D model 101, what the severity value of tooth wear is and/or what the exact location of the tooth wear lesion is, on the virtual 3D model 101.

There may be various ways of displaying the at least part of the virtual 3D model 101 with registered tooth wear. For example, color, transparency, zoom level and/or shading of the at least part of the virtual 3D model 101 may be varied with respect to the rest of the virtual 3D model 101.

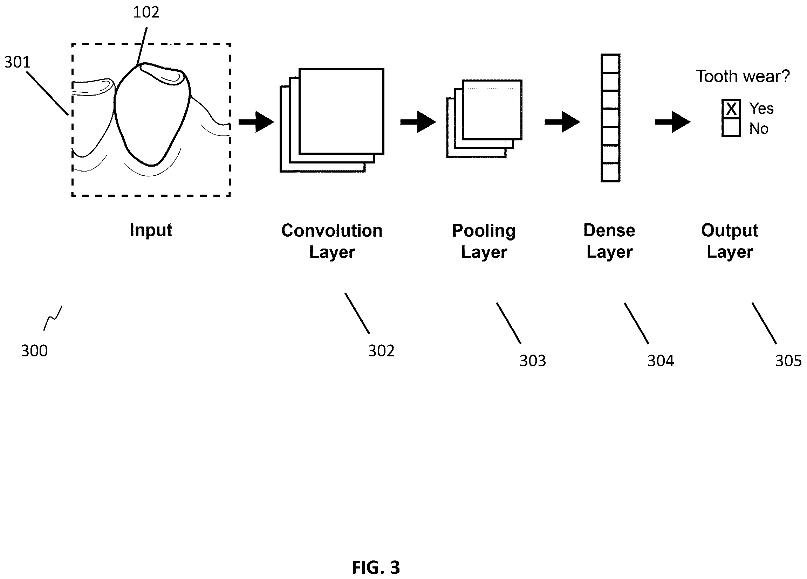

Figure 3 illustrates a trained neural network 300 used in the method according to an embodiment. The trained neural network 300 in this case may be a convolutional neural network and may comprise a plurality of convolution layers 302 capable of capturing low-level features of the input, i.e. obtaining convolved features. Moreover, one or more of pooling layers 303 may be present, responsible for reducing the spatial size of convolved features. Convolutional neural networks may be particularly suitable for processing matrix information such as 2D images.

The input 301 may be in a 2D or 3D format and may comprise the at least one input element. Figure 3 illustrates the input in the 2D format. The at least one input element may be a 2D image of a tooth 102 which has its occlusal surface affected by tooth wear. The at least one input element may alternatively comprise one or more of 2D images of the occlusal surface of the tooth 102 or at least one pixel of the 2D image of the tooth 102.

The output 305 may comprise the at least one probability value representing the likelihood that the at least one input element corresponds to the tooth wear class.

In case of FIG. 3, the tooth wear class is the indication of tooth wear presence on the at least one input element of the input 301. The trained neural network 300 in this case may perform a classification task to classify the input 301 into "Yes" or "No" output class. Alternatively or additionally, the tooth wear class may comprise the tooth wear severity value associated with tooth wear of the tooth 102. For example, the tooth wear severity value associated with tooth wear of the tooth 102 may state "severe", where the "severe" value may correspond to Tooth Wear Index (TWI) value 4.

A training process for the classification task of the trained neural network 300 may be described as follows. The overall aim of a non-trained neural network may be to develop the trained neural network 300 capable of classifying the input according to a desired tooth wear class. The tooth wear class may be the indication of tooth wear presence, the tooth wear severity value and/or the tooth wear type.

The training process may be referred to as "supervised training" due to use of a training dataset that has been labeled. The training dataset may be in 2D format, i.e. it may comprise a plurality of 2D training images of teeth or parts of teeth. Additionally, the training dataset may comprise associated labels where the labels correspond to the tooth wear class. The labeling may be performed for example by a qualified dental practitioner. The training dataset may be labeled according to the indication of tooth wear presence, for example with "Yes" or "No" labels indicating tooth wear presence or absence, respectively. Additionally or alternatively, the training dataset may be labeled with tooth wear severity labels and/or tooth wear type labels associated with corresponding tooth wear state.

The labels may be regarded as a target or a ground truth for a given training data set.

The training dataset may be fed into the non-trained neural network. Output of the non-trained neural network may be a probability value indicating which output class the input, or an input element, belongs to. An error may be determined between the ground truth and the output. This error may also be regarded as a loss function. One example of such a loss function may be a mean-squared-error function. In order to train the non-trained neural network, this loss function should be minimized. The minimization of the loss function may be achieved for example by using a stochastic gradient descent algorithm. Through this process of minimizing the loss function, weights of the non-trained neural network may be updated.

The training process may be an iterative process in which the same training data set may be fed, iteratively, into the non-trained neural network until a stopping criterion is reached. The stopping criterion may be a specific threshold value for the loss function. The stopping criterion may be a number of epochs (training cycles) after which performance metrics cease to increase.

The training dataset may alternatively be in 3D format comprising a plurality of teeth represented as 3D meshes, 3D graphs or point clouds. The tooth wear class may be the indication of tooth wear presence, the tooth wear severity value and/or the tooth wear type.

Regardless of the format of the training dataset, the training dataset may comprise geometric information associated with teeth within, or parts of teeth within. Similar to the input element of the input to the trained neural network, the geometric information of the training dataset may comprise a curvature information, a facet normal information, a depth information relating to a virtual camera position and/or an angle between a facet normal and a virtual camera direction relating to the teeth within, or parts of teeth within.

The curvature information of the training dataset may be relevant for successful training process of the non-trained neural network. For example, an abrupt change of relative curvature between areas in teeth surfaces in the training dataset may represent tooth wear.

The depth information of the training dataset may be relevant for the training process because information on tooth surface topology, of teeth from the training dataset, may be comprised within.

The facet normal information of the training dataset may be relevant for the training process. Neighboring facets of a tooth from the training dataset may have a pronounced difference between their facet normals, indicating tooth wear due to abrupt topological change.

The training dataset may comprise color information of the teeth or parts of teeth within. Severe stages of tooth wear may be characterized by a loss of tooth substance called enamel such that dentin gets exposed. Difference in colors between a white tone of enamel and a yellow color of the dentin is a strong indicator of severe tooth wear. Thus, this information may be highly relevant in the training process of the non-trained neural network for detecting tooth wear.

The training dataset may optionally comprise semantic information associated with the teeth or parts of teeth within. This type of information aids the training process because the non-trained neural network may learn how to process visual geometric information in an improved manner, by having semantic context added to the geometric information.

Training process for the segmentation task may the same as the training process for the classification task.

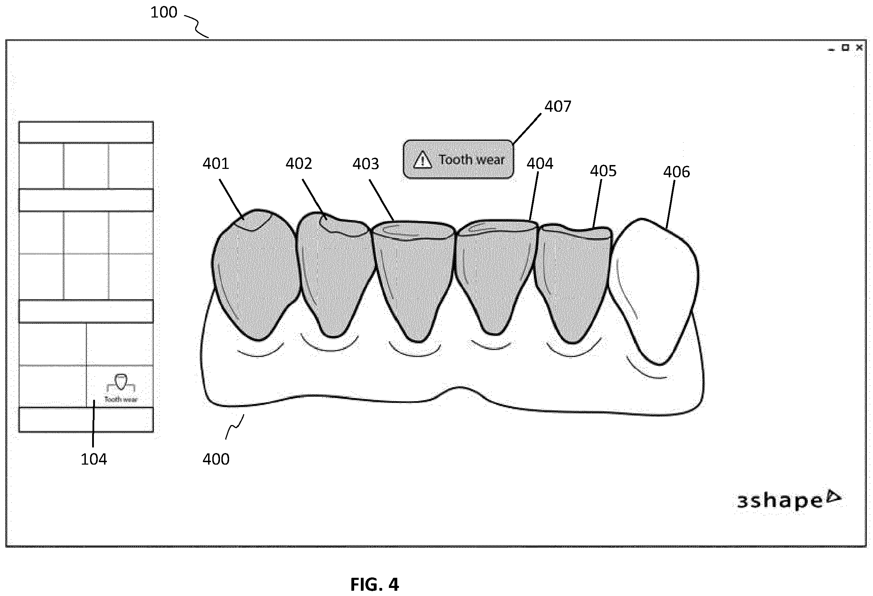

Figure 4 illustrates the displayed part 400 of the virtual 3D model 101 with highlighted multiple teeth 401-405 affected by tooth wear, according to an embodiment of invention. The part 400 of the virtual 3D model 101 may be a part of the virtual 3D model 101, for example a part of a lower jaw of the virtual 3D model 101. The tooth wear class, in case of FIG. 4, may be the indication of tooth wear presence. The tooth wear presence may be in form of a visual notification 407. Additionally, the tooth wear presence may be signaled by displaying teeth 401-405, affected by tooth wear, in a different color compared to tooth 406 not affected by tooth wear. The indication of tooth wear presence may additionally comprise an audio signal.

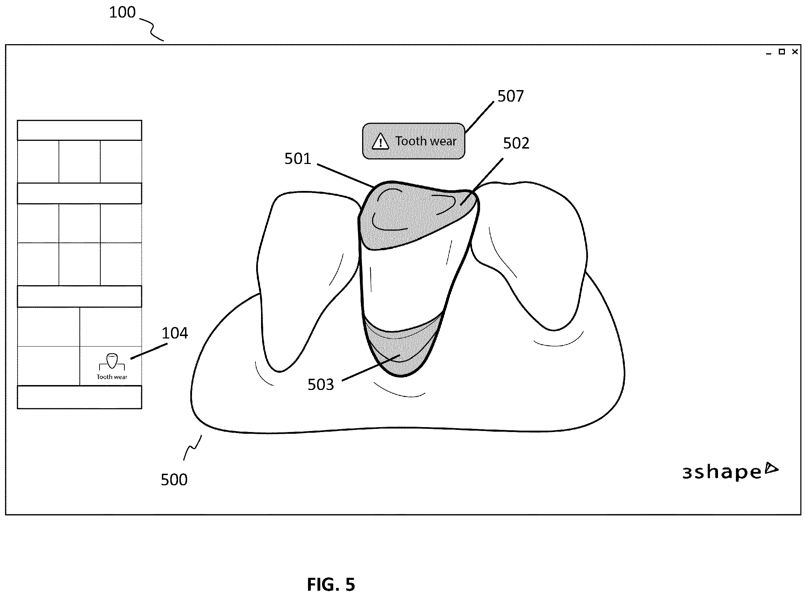

FIG. 5 illustrates a displayed part of the virtual 3D model with highlighted tooth wear on two tooth surfaces of a single tooth according to an embodiment of invention.

Figure 5 illustrates a displayed part 500 of the virtual 3D model 101 with highlighted tooth wear on two tooth surfaces 502, 503 of the tooth 501, according to an embodiment of invention.

The tooth wear class, in case of FIG. 5, may be the indication of tooth wear presence on tooth surfaces 502, 503 of the tooth 501 belonging to the part 500 of the virtual 3D model 101. As visible in FIG. 5, the cervical surface 503 and the occlusal surface 502 are affected by tooth wear. These surfaces may be displayed in a different color compared to teeth not affected by tooth wear. The indication on tooth wear presence may additionally comprise the visual notification 507.

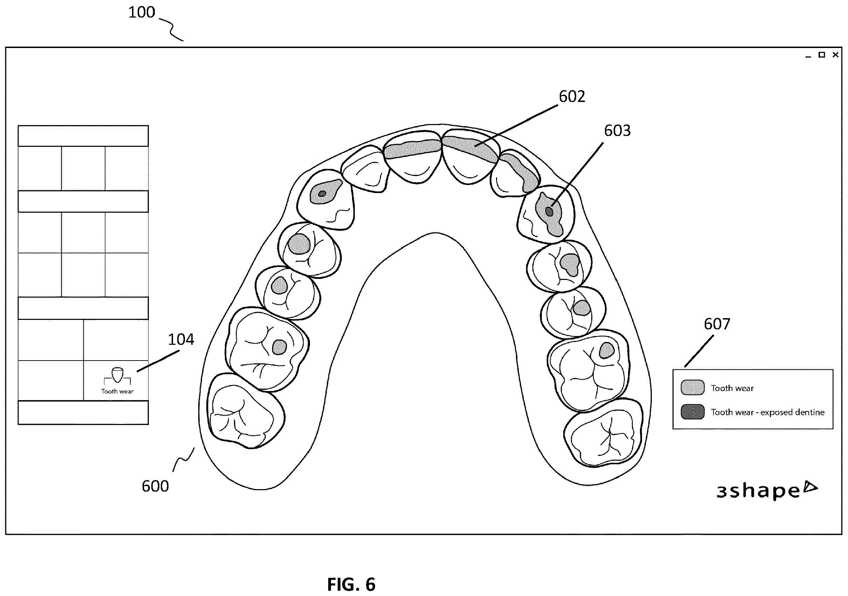

Figure 6 illustrates a displayed part 600 of the virtual 3D model 101 with highlighted tooth wear lesions 602, 603 on multiple teeth according to an embodiment of invention. Tooth wear, in case of FIG. 6, may show specific tooth wear lesions 602, 603 on the part 600 of the virtual 3D model 101. In contrast, FIG. 4 and FIG. 5 show highlighted entire tooth or tooth surface(s) affected by tooth wear. The tooth wear class, in the case of FIG. 6, may additionally comprise the tooth wear severity value 607. The three tooth wear severity values may be "No tooth wear", "Moderate tooth wear", "Severe tooth wear" that may be associated to teeth or tooth wear lesions on the teeth. Teeth or tooth wear lesions associated with one value of tooth wear severity values may be colored differently compared to teeth or tooth wear lesions associated with another tooth wear severity value.

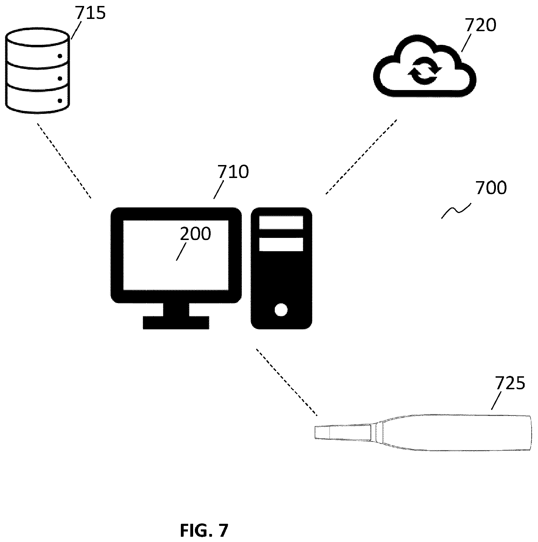

Figure 7 illustrates a dental scanning system 700 which may comprise a computer 710 capable of carrying out the method according to any one or more embodiments of invention, for example according to the method 200. The computer may comprise a wired or a wireless interface to a server 715, a cloud server 720 and an intraoral scanner 725. The intraoral scanner 725 may be capable of recording scan data of the patient's dentition.

The dental scanning system 700 may comprise a processor configured to carry out the method according to one or more embodiments of the disclosure. The processor may be a part of the computer 710, the server 715 or the cloud server 720.

A non-transitory computer-readable storage medium may be comprised in the dental scanning system 700. The non-transitory computer-readable medium can carry instructions which, when executed by a computer, cause the computer to carry out the method according to one or more embodiments of the disclosure.

A computer program product may be embodied in the non-transitory computer-readable storage medium. The computer program product may comprise instructions which, when executed by the computer, cause the computer to perform the method according to any of the embodiments presented herein.

Although some embodiments have been described and shown in detail, the disclosure is not restricted to such details, but may also be embodied in other ways within the scope of the subject-matter defined in the following claims. In particular, it is to be understood that other embodiments may be utilized, and structural and functional modifications may be made without departing from the scope of the present invention.

It should be appreciated that reference throughout this specification to "one embodiment" or "an embodiment" or "an aspect" or features included as "may" means that a particular feature, structure or characteristic described in connection with the embodiments is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

## Claims

1. A computer-implemented method for detecting tooth wear on a virtual 3D model of a dental situation, the method comprising:
- receiving, by a processor, the virtual 3D model of the dental situation;
- generating an input, from the virtual 3D model, for a trained neural network for tooth wear detection, wherein the input comprises at least one input element corresponding to at least a part of the virtual 3D model, the at least one input element comprising geometric information of the at least part of the virtual 3D model;
- providing the input to the trained neural network;
- obtaining an output from the trained neural network based on the provided input, wherein the output comprises at least one probability value, wherein the at least one probability value represents a likelihood that the at least one input element corresponds to a tooth wear class;
- assigning the tooth wear class to the at least one input element;
- registering tooth wear on the at least part of the virtual 3D model based on the assigned tooth wear class to the at least one input element;
- displaying the at least part of the virtual 3D model with the registered tooth wear.

2. The method according to the previous claim 1, wherein generating the input comprises generating a point cloud representation of the virtual 3D model.

3. The method according to the previous claim 2, wherein the at least one input element is a point from the point cloud representation of the virtual 3D model.

4. The method according to the previous claim 1, wherein generating the input comprises generating one or more 2D images of at least one tooth surface of at least one tooth in the virtual 3D model.

5. The method according to the previous claim 4, wherein the at least one tooth in the virtual 3D model is obtained by segmenting the virtual 3D model into a plurality of teeth.

6. The method according to previous claim 4 or 5, wherein the at least one input element is a pixel from the one or more 2D images.

7. The method according to any previous claim, wherein the geometric information of the at least part of the virtual 3D model is a curvature information, a facet normal information, a depth information relating to a virtual camera position and/or an angle between a facet normal and a virtual camera direction.

8. The method according to any previous claim, wherein the tooth wear class indicates presence of tooth wear on the at least one input element.

9. The method according to any previous claim 2-7, wherein the tooth wear class is a tooth wear severity value.

10. The method according to any previous claim 2-7, wherein the tooth wear class is a tooth wear type.

11. The method according to any previous claim, further comprising determining a distribution of the tooth wear class in the input and selecting outliers.

12. The method according to the previous claim, further comprising identifying a tooth wear indication based on the selected outliers and displaying the tooth wear indication.

13. The method according to any previous claim, wherein the input further comprises semantic information of the virtual 3D model, wherein semantic information comprises a tooth identifier, a tooth surface identifier and/or a jaw identifier.

14. A computer program product comprising instructions which, when the program is executed by a computer, causes the computer to carry out the method of any one of claims 1-13.

15. A non-transitory computer readable medium comprising instructions which, when executed by a computer, cause the computer to carry out the method of any of claims 1-13.
